# FASCICULE DE BREVET EUROPEEN

(11) **EP 2 892 874 B1**
(45) Date de publication et mention de la délivrance du brevet: **01.02.2017**
(21) Numéro de dépôt: 13762092.8
(22) Date de dépôt: 09.09.2013
(51) Int. Cl.: C07C 209/52, C07C 211/30, C07C 249/02, C07C 251/16, C07F 9/40, A61K 31/135, A61P 5/18

(54) **PROCEDE DE PREPARATION DU CINACALCET**
VERFAHREN ZUR HERSTELLUNG VON CINACALCET
PROCESS FOR PREPARING CINACALCET

(30) Priorité: 07.09.2012 FR 1258408
(43) Date de publication de la demande: 15.07.2015
(73) Titulaire: Produits Chimiques Auxiliaires et de Synthese, 91160 Longjumeau (FR)
(72) Inventeur: SCHNEIDER, Jean-Marie, F-78200 Magnanville (FR); MADEC, Jonathan, F-78400 Chatou (FR); KREIMERMAN, Sergio, F-78550 Houdan (FR); GUILLAMOT, Gérard, F-78220 Viroflay (FR)
(74) Mandataire: Regimbeau
(86) Numéro de dépôt international: PCT/EP2013/068617
(87) Numéro de publication internationale: WO 2014/037563

(56) Documents cités:
- WO-A1-2009/153814
- US-A1- 2009 093 652

## Description

La présente invention concerne un procédé de préparation du cinacalcet ((R)-α-méthyle-N-[3-[3-(trifluorométhyle)phényle]-propyle]-1-naphtalèneméthanamine) ou un de ses sels pharmaceutiquement acceptables faisant intervenir notamment comme intermédiaire de synthèse le ((R)-1-(1-naphthyle)ethylamino)vinyle phosphonate de diéthyle.

Le cinacalcet est un principe actif calcimimétique utilisé dans le traitement de l'hyperparathyroïdie et commercialisé notamment sous le nom Sensipar^{®}.

Le cinacalcet a été décrit pour la première fois en 2006 dans le brevet US 6,211,244. Bien qu'un procédé pour sa préparation ne soit pas décrit dans ce brevet, des composés analogues ont été préparés par réaction entre une amine et un aldéhyde suivie d'une réduction de l'imine obtenue en présence d'un cyanoborohydrure.

Des procédés de préparation du cinacalcet, de composés analogues et de leurs sels pharmaceutiquement acceptables ont été décrits dans les documents US 6,211,244, US 7,250,533, US 5,648,541, US 7,247,751, US 7,393,967, WO 06/125026, WO 06/127933, WO 06/127941, WO 07/062147, WO 07/112280, WO 07/127445, WO 07/127449, WO 08/058235, WO 08/000423, WO 08/035212, WO 08/058236, WO 08/06862, WO 2009153814, WO 2010067204, WO 2010015935,

WO 2010049293, WO 2010103531, WO 2010128388, WO 2011050499 et US 2009/093652.

WO 2010049293, WO 2010103531, WO 2010128388, WO 2011050499 et

WO 2009/153814 décrit un procédé de préparation du cinacalcet selon le schéma réactionnel suivant :

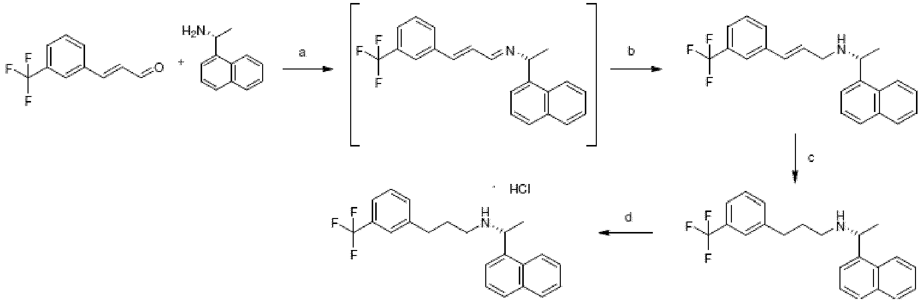

Dans ce procédé, une imine insaturée, préparée à partir de la (R)-(+)-1-(1-naphtyl)éthylamine et du 3-[3-(trifluorométhyl)phényl]propènaldéhyde, est transformée en cinacalcet insaturé sous forme libre, qui est ensuite réduit par hydrogénation catalysée par le palladium et le cinacalcet sous forme libre est transformé en chlorhydrate de cinacalcet avec de l'acide chlorhydrique gazeux.

WO 2010/015935 décrit un autre procédé de préparation du cinacalcet selon le schéma réactionnel suivant : 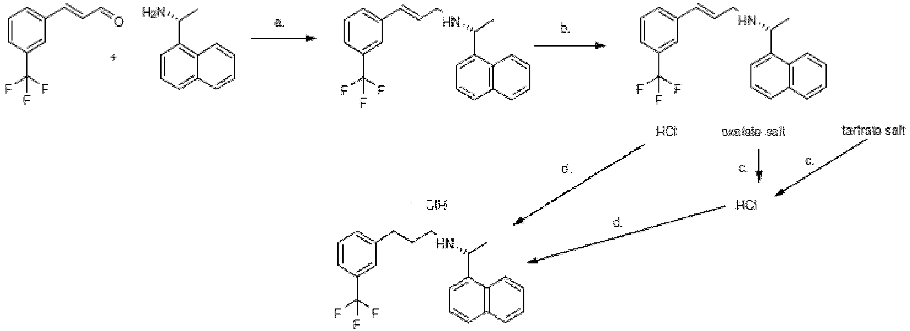 dans lequel : a. NaBH₄, méthanol, b. HCl, acétonitrile / acide oxalique, acétonitrile / acide di-*p*-toluoyl-L-tartrique, méthanol, c. (1) NaOH, eau (2) HCl, acétonitrile, d. (1) Na₂CO₃, eau, EtOAc (2) Pd(OH)₂, H₂ (3) NaHCO₃, di-*tert*-butyl dicarbonate, eau, tétrahydrofurane (4) HCl, méthanol.

Les procédés de synthèse décrits dans WO 2009/153814 et WO 2010/015935 ont les inconvénients de faire intervenir des borohydrures qui sont toxiques et de nécessiter des séquences réactionnelles compliquées pour purifier le cinacalcet.

De plus, la plupart de ces procédés nécessite l'utilisation d'intermédiaires complexes et de réactifs qui ne sont pas disponibles commercialement et doivent être synthétisés au préalable. Ces procédés ne sont donc pas adaptés à une application industrielle.

Il existe donc un réel besoin de développer une nouvelle voie de synthèse du cinacalcet ne présentant pas les inconvénients des procédés de l'art antérieur.

Les inventeurs ont ainsi développé un nouveau procédé de synthèse du cinacalcet qui permet d'avoir une synthèse plus courte et donc plus économique et plus performante.

La présente invention concerne donc un procédé de préparation du cinacalcet de formule (I) suivante : 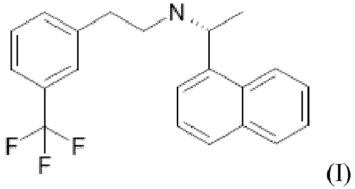 ou l'un de ses sels pharmaceutiquement acceptables comprenant la réaction du 3-(trifluorométhyl)benzaldéhyde de formule (II) suivante : 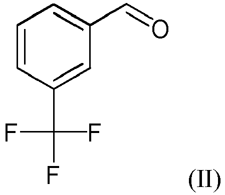 avec le dérivé phosphoré de formule (III) suivante : 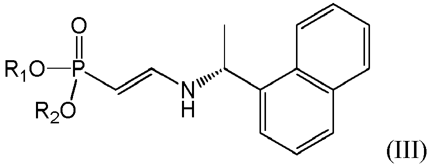 dans laquelle R₁ et R₂, identiques ou différents, de préférence identiques, représentent chacun un groupement (C₁-C₆)alkyle, tel qu'un groupe éthyle.

Dans la présente invention, on entend désigner par « pharmaceutiquement acceptable » ce qui est utile dans la préparation d'une composition pharmaceutique, qui est généralement sûre, non toxique et ni biologiquement ni autrement non souhaitable et qui est acceptable pour une utilisation vétérinaire de même que pharmaceutique humaine.

On entend désigner par « sels pharmaceutiquement acceptables » d'un composé, des sels qui sont pharmaceutiquement acceptables, comme défini ici, et qui possèdent l'activité pharmacologique souhaitée du composé parent. De tels sels comprennent :
(1) les sels d'addition d'acide pharmaceutiquement acceptable formés avec des acides inorganiques pharmaceutiquement acceptables tels que l'acide chlorhydrique, l'acide bromhydrique, l'acide sulfurique, l'acide nitrique, l'acide phosphorique et similaires ; ou formés avec des acides organiques pharmaceutiquement acceptables tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires, ou
(2) les sels d'addition de base pharmaceutiquement acceptable formés lorsqu'un proton acide présent dans le composé parent est soit remplacé par un ion métallique, par exemple un ion de métal alcalin, un ion de métal alcalino-terreux ou un ion d'aluminium ; soit coordonné avec une base organique ou inorganique pharmaceutiquement acceptable. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Avantageusement, il s'agira d'un sel d'addition d'acide tel qu'un chlorhydrate ou un phosphate.

Par groupement « (C₁-C₆)alkyle », on entend, au sens de la présente invention, une chaîne hydrocarbonée saturée, linéaire ou ramifiée, comportant 1 à 6, de préférence 1 à 4, atomes de carbone. A titre d'exemple, on peut citer les groupes méthyle, éthyle, propyle, isopropyle, butyle, isobutyle, sec-butyle, tert-butyle, pentyle ou encore hexyle.

Par « alkoxyde de métal alcalin », on entend, au sens de la présente invention, un composé de formule AlkOM dans laquelle M représente un métal alcalin tel que le sodium, le potassium ou le lithium, et de préférence le sodium ou le potassium, et Alk représente un groupe (C₁-C₆)alkyle tel que défini ci-dessus. Il pourra s'agir en particulier de *tert*-butoxyde de potassium ou de sodium.

La réaction entre les composés de formule (II) et (III) sera avantageusement réalisée en présence d'une base. La base pourra être choisie parmi l'hydrure de sodium, un alkoxyde de métal alcalin (par exemple le *tert*-butoxyde de potassium ou de sodium), le 2,2,6,6-tétraméthylpipéridide de lithium (LiTMP), l'hexaméthyldisilazide de lithium ou de potassium (LiHMDS ou KHMDS), le diisopropylamide de lithium (LDA) ou encore le 1,8-diazabicyclo[5.4.0]undéc-7-ène (DBU). La base préférée sera l'hydrure de sodium.

La réaction entre les composés de formule (II) et (III) pourra être réalisée dans un solvant. De préférence, la réaction sera effectuée dans un solvant aprotique polaire tel que le diméthylformamide (DMF), le diméthylsulfoxyde (DMSO), et les solvants éthérés.

Avantageusement, le solvant sera un solvant éthéré tel que le tétrahydrofurane (THF), le 2-méthyl-tétrahydrofurane (MeTHF) ou encore l'éther diméthylique (DME).

Le rapport entre la quantité molaire de l'aldéhyde de formule (II) et la quantité molaire du dérivé phosphoré de formule (III) sera avantageusement compris entre 0,9:1 et 1:0,9, notamment compris entre 0,95:1 et 1:0,95, de préférence sera d'environ 1:1 (quantités équimolaires des composés (II) et (III)).

La réaction entre les composés de formule (II) et (III) permet d'obtenir l'énamine de formule (IV) suivante : 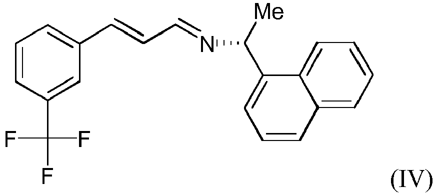 qui peut alors être hydrogénée pour donner le cinacalcet de formule (I), celui-ci étant en outre éventuellement converti en un sel pharmaceutiquement acceptable.

La réduction du composé de formule (IV) pourra être réalisée sous atmosphère d'hydrogène en présence d'un catalyseur d'hydrogénation notamment à base d'un métal. Le métal peut être par exemple le palladium, le nickel ou le rhodium.

Selon un mode préféré de réalisation, la réduction est réalisée avec du palladium sur charbon (Pd/C) sous atmosphère d'hydrogène.

La réduction peut être effectuée dans un solvant. On citera parmi les solvants appropriés les esters tels que les acétates de (C₁-C₆)alkyle, en particulier l'acétate d'éthyle, les (C₁-C₆)-alcanols (c'est-à-dire un composé de formule R-OH avec R = (C₁-C₆)alkyle) tels que l'éthanol ou le méthanol, et leurs mélanges. Le solvant préféré est l'éthanol.

Les conditions dans lesquelles la réduction peut être effectuée, telles que la température, la quantité de métal, la pression d'hydrogène, le temps de réaction et la concentration, peuvent être déterminées par l'homme du métier.

Avantageusement, la réduction est réalisée à une température entre 20 et 25°C en présence d'une quantité catalytique du catalyseur d'hydrogénation, avantageusement à base de palladium tel que Pd/C, et sous une pression d'hydrogène supérieure ou égale à 1 bar, notamment comprise entre 1 et 5 bars. La quantité de catalyseur est avantageusement inférieure à 10 % molaire par rapport à la quantité en moles du composé de formule (IV) à hydrogéner.

Le cinacalcet pourra être isolé après la réduction de l'énamine de formule (IV) sous sa forme libre ou sous la forme d'un sel selon des procédés bien connus de l'homme du métier.

De préférence, le cinacalcet sera obtenu par précipitation sous la forme d'un sel d'acide, pouvant être pharmaceutiquement acceptable, tel que le sel de phosphate ou le chlorhydrate du cinacalcet. Le sel de cinacalcet ainsi précipité pourra ensuite être converti en un autre sel de cinacalcet qui sera pharmaceutiquement acceptable.

Le dérivé phosphoré de formule (III) telle que définie précédemment pourra être obtenu par réaction du dérivé phosphonate de formule (V) suivante : 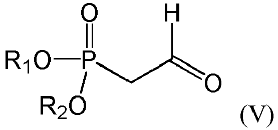 dans laquelle R₁ et R₂ sont tels que définis précédemment,
avec la (R)-(+)-1-(1-naphtyl)éthylamine de formule (VI) suivante :

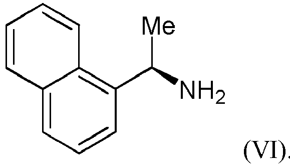

Cette réaction pourra être réalisée dans un solvant éthéré tel que le tétrahydrofurane (THF), le 2-méthyltétrahydrofurane (MeTHF) ou l'éther diméthylique (DME).

Le rapport entre la quantité molaire du dérivé phosphonate de formule (V) et la quantité molaire de l'amine (VI) sera avantageusement compris entre 0,9:1 et 1:0,9, notamment compris entre 0,95:1 et 1:0,95, de préférence sera d'environ 1:1 (quantités équimolaires des composés (V) et (VI)).

Dans un mode préféré de réalisation, la réaction entre le composé de formule (V) et le composé de formule (VI) pourra être réalisée à une température comprise entre 0 et 20°C, avantageusement d'environ 10°C.

Le dérivé phosphonate de formule (V) telle que définie précédemment pourra être obtenu par hydrolyse du composé de formule (VII) suivante : 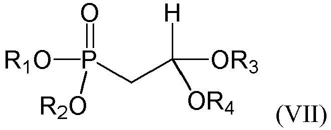 dans laquelle R₁ et R₂ sont tels que définis précédemment et R₃ et R₄, identiques ou différents, de préférence identiques, représentent un groupe (C₁-C₆)alkyle, tel qu'un groupe éthyle.

De préférence, le composé de formule (VII) sera le 2,2-diéthoxyéthylphosphonate de diéthyle.

Cette réaction d'hydrolyse sera avantageusement réalisée en milieu acide, notamment en présence d'acide oxalique ou d'acide chlorhydrique.

L'hydrolyse du composé de formule (VII) et la réaction du dérivé phosphoré de formule (V) avec l'amine de formule (VI) pourront être réalisées dans un seul et même réacteur sans isolement des intermédiaires de synthèse.

Le procédé selon l'invention permet ainsi d'obtenir le cinacalcet ou l'un de ses sels pharmaceutiquement acceptables selon le schéma réactionnel suivant : 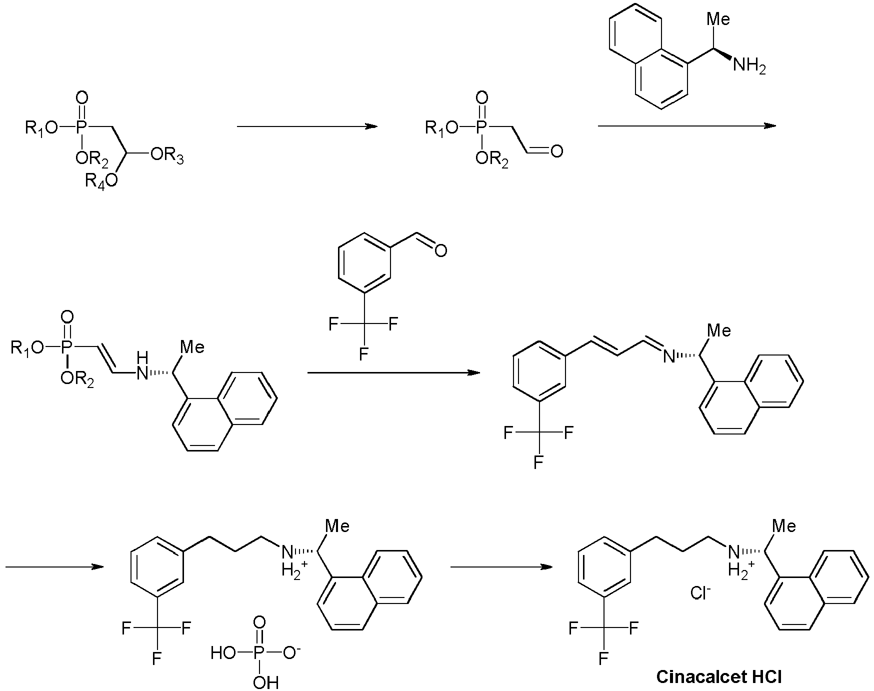 dans lequel R₁, R₂, R₃ et R₄ sont tels que définis précédemment.

Ce procédé présente l'avantage d'être réalisable en peu d'étapes, à partir de réactifs non toxiques, bons marchés et disponibles facilement dans le commerce.

La présente invention concerne également le (R)-(+)-1-(1-naphtyl)éthylamino-vinyl-dialkyl-phosphonate de formule (III) 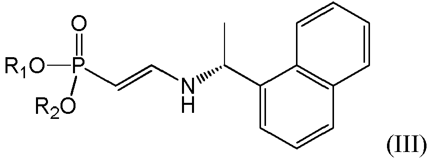 dans laquelle R₁ et R₂ sont tels que définis précédemment.

La présente invention concerne également l'utilisation d'un dérivé phosphoré de formule (III) telle que définie précédemment comme intermédiaire de synthèse dans un procédé de préparation du cinacalcet de formule (I) telle que définie précédemment ou d'un de ses sels pharmaceutiquement acceptables, et en particulier du chlorhydrate de cinacalcet.

La présente invention concerne également un procédé de préparation du dérivé phosphoré de formule (III) telle que définie précédemment comprenant la réaction du dérivé phosphonate de formule (V) suivante : 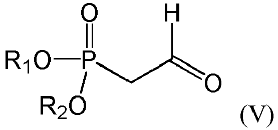 dans laquelle R₁ et R₂ sont tels que définis précédemment,
avec la (R)-(+)-1-(1-naphtyl)éthylamine de formule (VI) suivante :

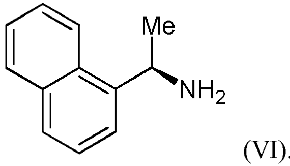

Cette réaction pourra être réalisée dans un solvant éthéré tel que le tétrahydrofurane (THF), le 2-méthyltétrahydrofurane (MeTHF) ou l'éther diméthylique (DME).

Le rapport entre la quantité molaire du dérivé phosphonate de formule (V) et la quantité molaire de l'amine (VI) sera avantageusement compris entre 0,9:1 et 1:0,9, notamment compris entre 0,95:1 et 1:0,95, de préférence sera d'environ 1:1 (quantités équimolaires des composés (V) et (VI)).

Dans un mode préféré de réalisation, la réaction entre le composé de formule (V) et le composé de formule (VI) pourra être réalisée à une température comprise entre 0 et 20°C, avantageusement d'environ 10°C.

Le dérivé phosphonate de formule (V) telle que définie ci-dessus pourra être obtenu par hydrolyse du composé de formule (VII) suivante : 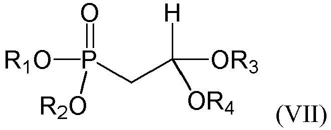 dans laquelle R₁ et R₂ sont tels que définis précédemment et R₃ et R₄, identiques ou différents, de préférence identiques, représentent un groupe (C₁-C₆)alkyle, tel qu'un groupe éthyle.

De préférence, le composé de formule (VII) sera le 2,2-diéthoxyéthylphosphonate de diéthyle.

Cette réaction d'hydrolyse sera avantageusement réalisée en milieu acide, notamment en présence d'acide oxalique ou d'acide chlorhydrique.

L'hydrolyse du composé de formule (VII) et la réaction du dérivé phosphoré de formule (V) avec l'amine de formule (VI) pourront être réalisées dans un seul et même réacteur sans isolement des intermédiaires de synthèse.

Un procédé d'obtention et/ou de purification d'une forme cristalline d'un sel pharmaceutiquement acceptable du cinacalcet, avantageusement à partir du cinacalcet obtenu par le procédé de préparation du cinacalcet de l'invention détaillé précédemment, est divulgué en particulier un procédé d'obtention du sel de phosphate du cinacalcet ou du chlorhydrate de cinacalcet.

Dans un premier mode de réalisation, la forme cristalline du sel pharmaceutiquement acceptable du cinacalcet sera obtenue par la neutralisation d'un sel du cinacalcet suivie de l'addition d'un acide pharmaceutiquement acceptable. Selon ce premier mode de réalisation, le chlorhydrate du cinacalcet peut être préparé à partir du sel de phosphate de cinacalcet.

Dans un second mode de réalisation, la forme cristalline du sel pharmaceutiquement acceptable du cinacalcet sera obtenue par recristallisation d'un sel pharmaceutiquement acceptable du cinacalcet, tel que le chlorhydrate de cinacalcet.

Dans un troisième mode de réalisation, la forme cristalline du sel pharmaceutiquement acceptable du cinacalcet sera obtenue par addition d'un acide pharmaceutiquement acceptable au cinacalcet sous forme de base libre et recristallisation du sel pharmaceutiquement acceptable de cinacalcet obtenu, tel que le chlorhydrate de cinacalcet.

Un procédé de préparation du chlorhydrate de cinacalcet est divulgué comprenant les étapes successives suivantes :
(a) neutralisation d'un sel de phosphate de cinacalcet pour donner le cinacalcet sous forme de base libre, et
(b) réaction du cinacalcet sous forme de base libre obtenu à l'étape (a) précédente avec de l'acide chlorhydrique, éventuellement sous forme gazeuse (encore appelé chlorure d'hydrogène), pour donner le chlorhydrate de cinacalcet.

Un tel procédé permet d'obtenir le chlorhydrate de cinacalcet sous une forme purifiée. Le choix du sel de phosphate dans ce procédé est important puisque l'utilisation d'autres sels ne permet pas d'obtenir le chlorhydrate de cinacalcet avec un aussi bon degré de pureté. De même, la conversion directe du cinacalcet sous forme de base libre en chlorhydrate de cinacalcet conduit à un produit qui doit être recristallisé plusieurs fois pour obtenir un degré de pureté acceptable.

### Etape (a) :

L'étape de neutralisation sera avantageusement réalisée dans l'acétate d'éthyle comme solvant.

Une base telle que l'hydroxyde de sodium, notamment en solution aqueuse, pourra être utilisée pour libérer le cinacalcet sous forme de base libre à partir de son sel de phosphate.

La neutralisation pourra ainsi être effectuée dans un mélange biphasique comprenant un solvant organique, tel que l'acétate d'éthyle, et une solution aqueuse contenant une base, telle qu'une solution aqueuse d'hydroxyde de sodium.

Une fois le cinacalcet sous forme de base libre obtenu, le mélange réactionnel pourra être lavé à l'eau, de préférence déminéralisée, afin d'éliminer tous les sels minéraux formés au cours de cette étape.

Si nécessaire, la quantité de solvant utilisée dans cette étape, et plus particulièrement d'acétate d'éthyle, pourra être ajustée avant de réaliser l'étape b) suivante soit en rajoutant du solvant, soit en en distillant une partie pour en diminuer le volume. Le solvant pourra également être complètement évaporé et être remplacé par un autre solvant. Avantageusement, une partie du solvant sera distillée avant d'engager l'étape b) dans le même solvant qui sera de préférence l'acétate d'éthyle.

### Etape (b) :

Cette étape pourra être avantageusement réalisée dans l'acétate d'éthyle comme solvant dans lequel le cinacalcet sous forme de base libre est solubilisé.

De l'acide chlorhydrique sous forme gazeuse ou éventuellement en solution dans l'acétate d'éthyle est ajouté au mélange réactionnel pour former le chlorhydrate de cinacalcet.

Une fois le chlorhydrate de cinacalcet formé, celui-ci peut être cristallisé par ajout d'un anti-solvant et/ou refroidissement du mélange réactionnel à une température inférieure ou égale à 30°C, de préférence inférieure comprise entre 0 et 30°C, notamment à une température d'environ 0°C.

Par « anti-solvant », on entend, au sens de la présente invention, un solvant dans lequel le chlorhydrate de cinacalcet n'est pas soluble, c'est-à-dire que sa solubilité dans ledit solvant est inférieure ou égale à 1 g/L. Il s'agira en particulier de l'heptane.

Le chlorhydrate de cinacalcet pourra ainsi être obtenu sous une forme cristalline et purifiée.

La forme cristalline du chlorhydrate de cinacalcet obtenu sera notamment la forme cristalline I telle que décrite dans la demande de brevet EP 1 883 618, qui est caractérisée par les pics suivants obtenus par diffractométrie des rayons X sur poudre : 13,9; 19,0; 21,3 ; et 25,5 ± 0,2° 2θ.

Le procédé de préparation du chlorhydrate de cinacalcet pourra ainsi comprendre les étapes successives suivantes :
(i) solubilisation d'un sel de phosphate de cinacalcet dans l'acétate d'éthyle,
(ii) ajout d'une base telle que l'hydroxyde de sodium, notamment en solution aqueuse afin de former le cinacalcet sous forme de base libre,
(iii) lavage du milieu réactionnel à l'eau, de préférence déminéralisée,
(iv) éventuellement distillation d'une partie du solvant,
(v) ajout d'acide chlorhydrique, notamment sous forme gazeuse ou éventuellement en solution dans l'acétate d'éthyle, afin de former le chlorhydrate de cinacalcet,
(vi) ajout d'heptane,
(vii) refroidissement du mélange réactionnel à une température inférieure ou égale à 30°C, de préférence inférieure comprise entre 0 et 30°C, notamment à une température d'environ 0°C, et
(viii) récupération du chlorhydrate de cinacalcet cristallisé ainsi formé, notamment par filtration.

Le sel de phosphate de cinacalcet pourra être sous une forme polymorphe caractérisée par les pics principaux suivants obtenus par diffraction des rayons X sur poudre :

| **Valeur d (Å)** | **Intensité relative (%)** |
|---|---|
| 19,86 | 100 |
| 9,95 | 12,9 |
| 6,62 | 36,7 |
| 6,29 | 14,1 |
| 6,02 | 10,8 |
| 5,62 | 15,4 |
| 5,24 | 12,8 |
| 5,04 | 16,0 |
| 4,87 | 7,2 |
| 4,72 | 8,4 |
| 4,53 | 10,1 |
| 4,37 | 20,1 |
| 4,29 | 15,5 |
| 4,18 | 24,3 |
| 3,97 | 12,9 |
| 3,84 | 13,1 |
| 3,56 | 14,8 |
| 3,49 | 10,2 |

Le sel de phosphate du cinacalcet est utilisé comme médicament, utilisé avantageusement dans le traitement de l'hyperparathyroïdie secondaire chez les patients présentant une insuffisance rénale chronique et de l'hypercalcémie chez les patients présentant un cancer de la glande parathyroïde.

Le sel de phosphate du cinacalcet est utilisé pour la préparation d'un médicament destiné en particulier au traitement de l'hyperparathyroïdie secondaire chez les patients présentant une insuffisance rénale chronique et de l'hypercalcémie chez les patients présentant un cancer de la glande parathyroïde.

Une méthode de traitement thérapeutique est divulguée, plus particulièrement en vue de traiter l'hyperparathyroïdie secondaire chez les patients présentant une insuffisance rénale chronique et l'hypercalcémie chez les patients présentant un cancer de la glande parathyroïde, comprenant l'administration à une personne qui en a besoin d'une quantité thérapeutiquement efficace de sel de phosphate du cinacalcet.

La présente demande divulgue une composition pharmaceutique comprenant du sel de phosphate de cinacalcet et un ou plusieurs excipients pharmaceutiquement acceptables. Cette composition pharmaceutique peut prendre la forme de toute forme de dosage appropriée correspondant à un mode d'administration approprié. La voie orale sous forme de gélule ou de comprimé sera privilégiée.

La présente invention sera mieux comprise à la lumière des exemples non limitatifs suivants.

### FIGURE

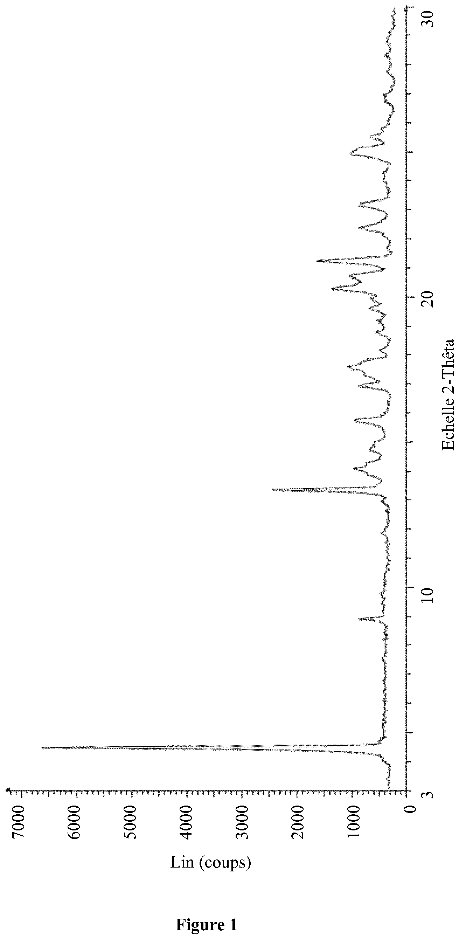

Figure 1 : Diagramme de diffraction des rayons X sur poudre du sel de phosphate de la (*R*)-(-)-α-méthyl-N-[3-[3-trifluorométhylphényl]propyl]-1-naphthalène-méthanamine.

### EXEMPLES

Le chlorhydrate du cinacalcet a été préparé selon le procédé de la présente invention comme représenté sur le schéma suivant :

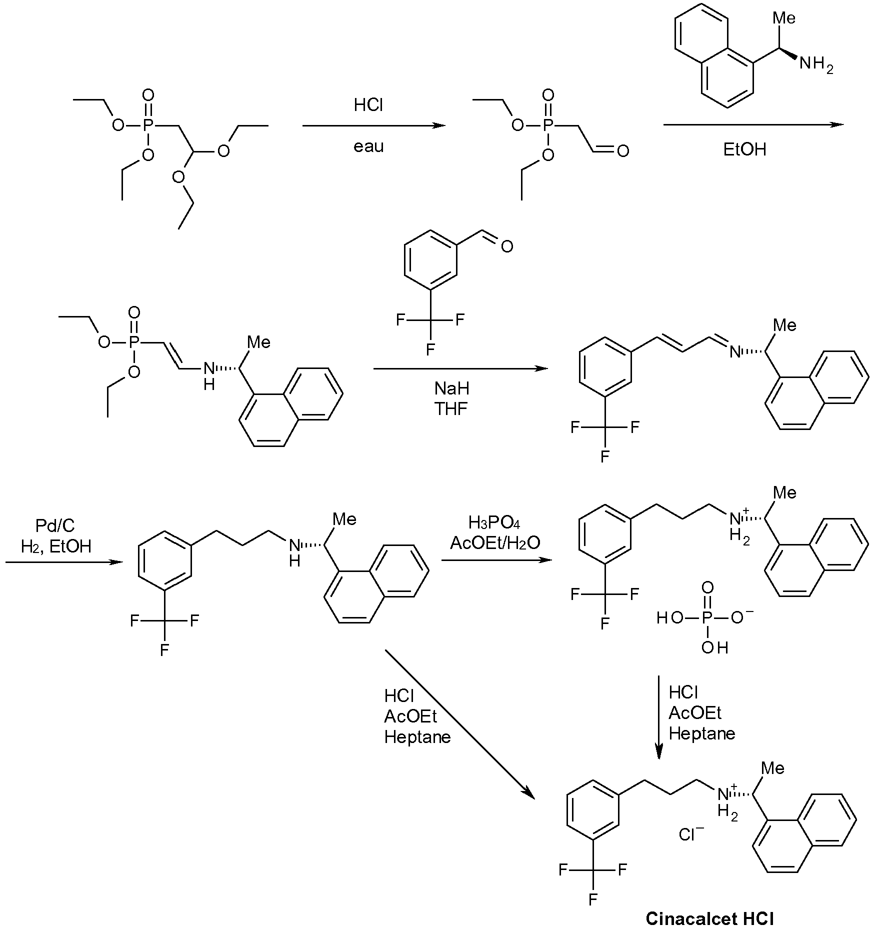

### Formylméthylphosphonate de diéthyle (Etape 1)

Dans un tétracol de 2 litres équipé d'un agitateur mécanique, d'une ampoule de coulée et d'une entrée d'azote, sont introduits le 2,2-diéthoxyéthylphosphonate de diéthyle (150 g) et de l'eau (300 mL), et un léger courant d'azote est passé en continu à travers le système. Le mélange résultant est chauffé à 50°C et de l'acide chlorhydrique (5,6 mL) est ajouté. Le mélange est alors agité pendant 4 heures, puis de l'eau est ajoutée et le mélange est concentré sous pression réduite. Du chlorure de sodium est ajouté au mélange résultant qui est ensuite extrait avec 4 portions d'acétate d'éthyle. Les phases organiques combinées sont distillées sous pression réduite.

### 2-((R)-1-(1-Naphthyl)éthylamino)vinylphosphonate de diéthyle (Etape 2)

Dans un tétracol de 1 litre équipé d'un agitateur mécanique, d'une ampoule de coulée et d'une entrée d'azote, sont introduits le formylméthylphosphonate de diéthyle (100 g) et de l'éthanol (500 mL). Sous atmosphère d'azote, le mélange résultant est refroidi à 10°C et la (*R*)-1-(1-naphthyl)éthylamine (85,6 g) est ajoutée sous agitation. Le mélange est agité pendant une heure additionnelle. Le solvant est distillé à partir de ce mélange sous pression réduite. Le résidu résultant (180 g) est utilisé dans l'étape suivante sans purification additionnelle.

### (R)-N-[3-[3-(Trifluorométhyl)phényl]-2-propénylimino]-N-[1-(1-naphthyl) éthylamine] (Etape 3)

Dans un tétracol de 1 litre équipé d'un agitateur mécanique, d'une ampoule de coulée et d'une entrée d'azote, sont introduits l'hydrure de sodium (10,72 g) (dispersion dans l'huile à 60 %) et le tétrahydrofurane (150 mL). Le système est mis sous azote et une solution de 2-((*R*)-1-(1-naphthyl)éthylamino)vinylphosphonate de diéthyle (74,3 g.) dans du tétrahydrofurane anhydre (220 mL) est ajouté. Le mélange est agité jusqu'à ce que la réaction soit complète. Une solution de 3-trifluorométhylbenzaldéhyde (38,8 g) est ajoutée et le mélange est agité pendant une heure additionnelle, puis de l'eau est ajoutée au mélange. La phase aqueuse est extraite avec de l'éther butylique méthylique. Les phases organiques combinées sont lavées avec de l'eau et distillées sous pression réduite. Le résidu résultant (78,8 g) est utilisé dans l'étape suivante sans purification additionnelle.

### 1^{ère} Alternative :

### Chlorhydrate de la (R)-(-)-α-méthyl-N-[3-[3-trifluorométhylphényl]propyl]-1-naphthalèneméthanamine (Etape 4)

Le catalyseur au palladium (Pd/C 5 % - 2,1 g) est ajouté à une solution de (R)-N-[3-[3-(trifluorométhyl)phényl]-2-propènylimino]-N-[1-(1-naphthyl)éthylamine] (44,2 g) dans l'éthanol (440 mL) à la température ambiante sous pression de dihydrogène (3 bar). Le mélange est agité jusqu'à ce que la réaction soit terminée. Le mélange hétérogène est filtré et une solution d'acide chlorhydrique à 20 % dans l'isopropanol (27,4 g) est ajoutée à la température ambiante. Le mélange est évaporé à sec sous pression réduite.

De l'acétate d'éthyle est ajouté et le mélange est agité. De l'heptane est ensuite ajouté à température ambiante puis le mélange est agité. Le solide obtenu est filtré, lavé et séché sous pression réduite pour donner 29,5 g de produit (rendement : 60 % ; pureté : 95,0 %).

Le produit est recristallisé une seconde fois selon la même procédure (traitement avec de l'acétate d'éthyle, suivi d'heptane) pour donner le produit désiré avec un rendement de 80 % et une pureté de 99,3 %.

### 2^{nde} Alternative :

### Sel de phosphate de la (R)-(-)-α-Méthyl-N-[3-[3-trifluorométhylphényl]propyl]-1-naphthalènemethanamine (Etape 4)

Dans un appareil à hydrogénation d'un litre muni d'un agitateur mécanique, le catalyseur au palladium (Pd/C 5 % - 4,8 g) et une solution de (R)-N-[3-[3-(trifluorométhyl)phényl]-2-propénylimino]-N-[1-(1-naphthyl)éthylamine] (82,2 g) dans l'éthanol (500 mL) à la température ambiante sous pression de dihydrogène (3 bars) sont agitées pendant 4 heures. Une fois que la réaction est terminée, le mélange hétérogène est filtré. Le solvant est distillé à partir du mélange sous pression réduite. De l'acétate d'éthyle est ajouté. Les solvants sont distillés à partir du mélange sous pression réduite. De l'acétate d'éthyle et de l'eau sont ajoutés à la température ambiante suivis d'acide phosphorique (85 % p/p - 19,3 mL) Après ensemencement (1 % wt/wt), le mélange est agité pendant 2 heures additionnelles à la température ambiante et refroidi à 0°C sur. Le mélange est agité à 0°C et le solide est filtré, lavé avec de l'acétate d'éthyle et séché sous pression réduite pour donner 63,5 g de produit (60 %).

Dans un tétracol de 1 litre équipé d'un agitateur mécanique et d'une entrée d'azote, sont introduits le sel de phosphate de la (*R*)-(-)-α-méthyl-N-[3-[3-trifluorométhylphényl] propyl]-1-naphthalèneméthanamine (100,0 g), de l'acétate d'éthyle (500 mL) et de l'eau (100 mL). La suspension résultante est chauffée à 70°C et après refroidie. Le solide obtenu est filtré, lavé à l'acétate d'éthyle et séché sous pression réduite pour donner 95,0 g de produit (95 %).

Le sel de phosphate ainsi obtenu a été analysé par diffraction des rayons X sur poudre (XRPD) dans les conditions suivantes :
- Diffractomètre Siemens D5000
- Anticathode cuivre, voltage 10 kV, intensité 40 mA
- Montage θ-θ, échantillon fixe
- Température ambiante
- Domaine de mesure : 3° à 30°
- Incrémentation entre chaque mesure : 0,04°
- Temps de mesure par pas : 4s
- Fentes fixes : 1,6 mm
- Pas de référence interne
- Procédure de zéro avec les fentes Siemens
- Données expérimentales obtenues traitées avec le logiciel EVA (v. 12.0)

Le diagramme de diffraction des rayons X sur poudre obtenu est présenté sur la Figure 1. Les pics principaux obtenus sur ce diagramme sont caractérisés dans le tableau ci-dessous.

| **Angle 20 (°)** | **Valeur d (Å)** | **Intensité (coups)** | **Intensité relative (%)** |
|---|---|---|---|
| 4,445 | 19,86 | 6655 | 100 |
| 8,880 | 9,95 | 856 | 12,9 |
| 13,356 | 6,62 | 2444 | 36,7 |
| 14,073 | 6,29 | 941 | 14,1 |
| 14,714 | 6,02 | 716 | 10,8 |
| 15,765 | 5,62 | 1026 | 15,4 |
| 16,918 | 5,24 | 851 | 12,8 |
| 17,596 | 5,04 | 1068 | 16,0 |
| 18,200 | 4,87 | 476 | 7,2 |
| 18,787 | 4,72 | 561 | 8,4 |
| 19,600 | 4,53 | 669 | 10,1 |
| 20,306 | 4,37 | 1338 | 20,1 |
| 20,699 | 4,29 | 1030 | 15,5 |
| 21,239 | 4,18 | 1619 | 24,3 |
| 22,393 | 3,97 | 857 | 12,9 |
| 23,166 | 3,84 | 871 | 13,1 |
| 25,024 | 3,56 | 986 | 14,8 |
| 25,508 | 3,49 | 680 | 10,2 |

### Chlorhydrate de la (R)-(-)-α-méthyl-N-[3-[3-trifluorométhylphényl]propyl]-1-naphthalèneméthanamine (Etape 5)

Dans un tétracol de 1 litre équipé d'un agitateur mécanique, d'une ampoule de coulée et d'une entrée d'azote, sont introduits le sel de phosphate de la (*R*)-(-)-α-méthyl-N-[3-[3-trifluorométhylphényl]propyl]-l-naphthalèneméthanamine (92 g) et de l'acétate d'éthyle (368 mL) à température ambiante. Le système est mis sous azote et une solution d'hydroxyde de sodium (NaOH 20 % - 68 mL) dans l'eau (278 mL) est ajoutée sous agitation. La solution biphasique est agitée pendant 1 heure additionnelle. La phase organique est récupérée, lavée avec de l'eau, concentrés sous pression réduite et engagée pour la suite. Du gaz HCl (m g) est ajouté à la solution sous agitation et à température ambiante mais avec contrôle de la température pour faire en sorte que la température de la solution ne dépasse pas 25°C. La solution est refroidie à 10°C et agité pendant 30 minutes.

De l'heptane est ensuite ajouté puis le mélange est agité. La suspension résultante est agitée à 10°C puis est refroidie à 0°C. Le solide obtenu est filtré, lavé avec un mélange acétate d'éthyle / heptane 50:50 v/v et séché sous pression réduite pour donner 75,6 g du produit désiré (rendement : 94 % ; pureté : 99,9 %).

## Revendications

1. Procédé de préparation du cinacalcet ou d'un de ses sels pharmaceutiquement acceptables comprenant la réaction du 3-trifluorométhyl-benzaldéhyde de formule (II) suivante : 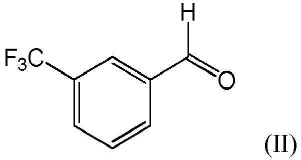 avec le dérivé phosphoré de formule (III) suivante : 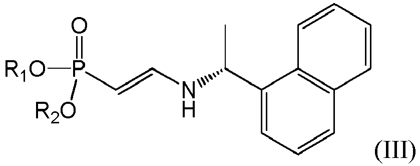 dans laquelle R₁ et R₂, identiques ou différents, de préférence identiques, représentent chacun un groupe (C₁-C₆)alkyle, tel qu'un groupe éthyle.

2. Procédé selon la revendication 1, **caractérisé en ce que** la réaction est réalisée en présence d'une base, de préférence choisie dans le groupe constitué par l'hydrure de sodium, un alkoxyde de métal alcalin (par exemple le *tert*-butoxyde de potassium ou de sodium), le 2,2,6,6-tétraméthylpipéridide de lithium (LiTMP), l'hexaméthyldisilazide de lithium ou de potassium (LiHMDS ou KHMDS), le diisopropylamide de lithium (LDA) et le 1,8-diazabicyclo[5.4.0]undéc-7-ène (DBU).

3. Procédé selon l'une quelconque des revendications 1 et 2, **caractérisé en ce que** la réaction est réalisée dans un solvant choisi parmi le diméthylformamide (DMF), le diméthylsufoxyde (DMSO), le tétrahydrofurane (THF), le 2-méthyl-tétrahydrofurane (MeTHF) et l'éther diméthylique (DME).

4. Procédé selon l'une quelconque des revendications 1 à 3, **caractérisé par** un rapport en poids (II):(III) d'environ 1:1.

5. Procédé selon l'une quelconque des revendications 1 à 4, **caractérisé en ce que** le composé de formule (IV) suivante : 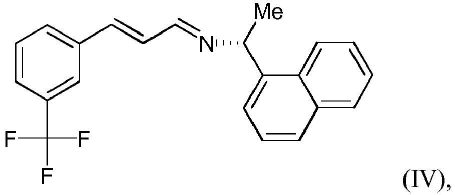 obtenu par réaction des composés de formule (II) et (III),
est hydrogéné pour donner le cinacalcet, celui-ci étant en outre éventuellement converti en un sel pharmaceutiquement acceptable.

6. Procédé selon la revendication 5, **caractérisé en ce que** l'hydrogénation est réalisée sous atmosphère d'hydrogène, en présence d'un catalyseur d'hydrogénation.

7. Procédé selon la revendication 6, **caractérisé en ce que** le catalyseur d'hydrogénation est du palladium sur charbon (Pd/C).

8. Procédé selon l'une quelconque des revendications 6 et 7, **caractérisé en ce que** la réaction d'hydrogénation est réalisée dans l'éthanol ou le méthanol comme solvant.

9. Procédé selon l'une quelconque des revendications 1 à 8, **caractérisé en ce que** le sel pharmaceutiquement acceptable de cinacalcet est le chlorhydrate de cinacalcet ou le sel de phosphate de cinacalcet.

10. Dérivé phosphoré de formule (III) suivante : 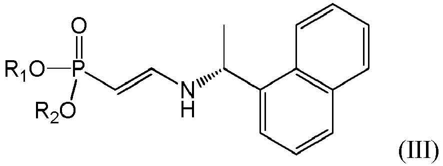 dans laquelle R₁ et R₂ sont tels que définis à la revendication 1.

11. Utilisation d'un dérivé phosphoré de formule (III) selon la revendication 10 comme intermédiaire de synthèse dans un procédé de préparation du cinacalcet ou d'un de ses sels pharmaceutiquement acceptables.

12. Procédé de préparation d'un dérivé phosphoré de formule (III) selon la revendication 10 comprenant la réaction du dérivé phosphonate de formule (V) suivante : 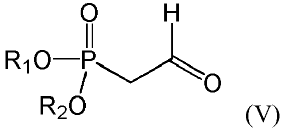 dans laquelle R₁ et R₂ sont tels que définis à la revendication 1,
avec la (R)-(+)-1-(1-naphtyl)éthylamine de formule (VI) suivante :

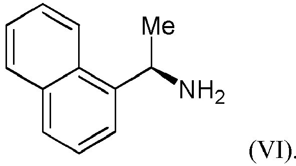

## Patentansprüche

1. Verfahren zur Herstellung von Cinacalcet oder eines seiner pharmazeutisch verträglichen Salze, die Reaktion des 3-Trifluormethyl-Benzaldehyd der folgenden Formel (II) umfassend: 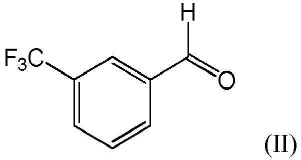 mit dem Phosphorderivat der folgenden Formel (III) : 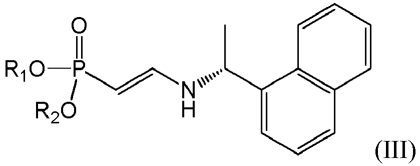 wobei identische oder unterschiedliche, vorzugsweise identische R₁ und R₂ jeweils eine (C₁-C₆)Alkylgruppe, wie eine Ethylgruppe, repräsentieren.

2. Verfahren nach Anspruch 1, **dadurch gekennzeichnet, dass** die Reaktion im Beisein einer Base durchgeführt wird, die vorzugsweise aus der Gruppe ausgewählt wird, die durch Natriumhydrid, einem Alkalimetallalkoxid (zum Beispiel Kalium- oder Natrium*tert*-Butoxyd), Lithium-2,2,6,6-Tetramethylpiperidid (LiTMP), Lithium- oder Kalium-Hexamethyldisilazid (LiHMDS oder KHMDS), Lithium-Diisopropylamid (LDA) und 1,8-Diazabicyclo[5.4.0]undec-7-en (DBU) gebildet wird.

3. Verfahren nach irgendeinem der Ansprüche 1 und 2, **dadurch gekennzeichnet, dass** die Reaktion in einem Lösungsmittel durchgeführt wird, das aus Dimethylformamid (DMF), Dimethylsufoxyd (DMSO), Tetrahydrofuran (THF), 2-Methyltetrahydrofuran (MeTHF) und Dimethylether (DME) ausgewählt wird.

4. Verfahren nach einem der Ansprüche 1 bis 3, **gekennzeichnet durch** ein Gewichtsverhältnis (II):(III) von etwa 1:1.

5. Verfahren nach einem der Ansprüche 1 bis 4, **dadurch gekennzeichnet, dass** die Zusammensetzung der folgenden Formel (IV): 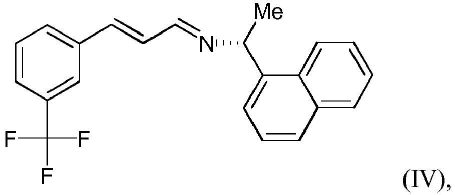 die man durch die Reaktion der Zusammensetzungen der Formel (II) und (III) erhält,
hydriert wird, um das Cinacalcet zu ergeben, wobei dieses darüber hinaus eventuell in ein pharmazeutisch verträgliches Salz umgewandelt wird.

6. Verfahren nach Anspruch 5, **dadurch gekennzeichnet, dass** die Hydrierung unter einer Wasserstoffatmosphäre im Beisein eines Hydrierkatalysators durchgeführt wird.

7. Verfahren nach Anspruch 6, **dadurch gekennzeichnet, dass** der Hydrierkatalysator Palladium auf Aktivkohle (Pd/C) ist.

8. Verfahren nach einem der Ansprüche 6 und 7, **dadurch gekennzeichnet, dass** die Hydrierungsreaktion im Ethanol oder Methanol als Lösungsmittel durchgeführt wird.

9. Verfahren nach einem der Ansprüche 1 bis 8, **dadurch gekennzeichnet, dass** das pharmazeutisch verträgliche Cinacalcet-Salz Cinacalcet-Chlorhydrat oder das Phosphat-Salz von Cinacalcet ist.

10. Phosphorderivat der folgenden Formel (III): 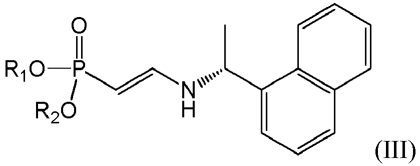 wobei R₁ und R₂ wie in Anspruch 1 definiert sind.

11. Verwendung eines Phosphorderivats der Formel (III) nach Anspruch 10 als Synthesezwischenprodukt in einem Verfahren zur Herstellung von Cinacalcet oder eines seiner pharmazeutisch verträglichen Salze.

12. Verfahren zur Herstellung eines Phosphorderivats der Formel (III) nach Anspruch 10, die Reaktion des Phosphonatderivats der folgenden Formel (V) umfassend: 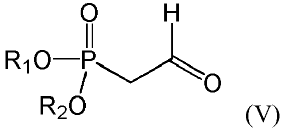 wobei R₁ und R₂ wie in Anspruch 1 definiert sind,
mit (R)-(+)-1-(1-Naphtyl)Ethylamin der folgenden Formel (VI):

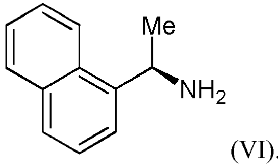

## Claims

1. A process for preparing cinacalcet or one of the pharmaceutically acceptable salts thereof, comprising the reaction of 3-trifluoromethyl-benzaldehyde of following formula (II): 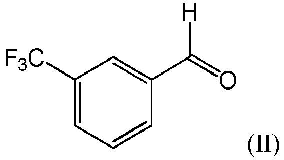 with the phosphorus-containing derivative of following formula (III): 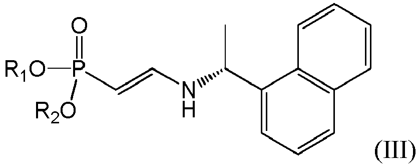 where R₁ and R₂, the same or different, preferably the same, each represent a (C₁-C₆)alkyl group such as an ethyl group.

2. The process according to claim 1, **characterized in that** the reaction is conducted in the presence of a base, preferably selected from the group formed by sodium hydride, an alkaline metal alkoxide (for example potassium or sodium *tert*-butoxide)), lithium 2,2,6,6-tetramethylpiperidide (LiTMP), lithium or potassium hexamethyldisilazide (LiHMDS or KHMDS), lithium diisopropylamide (LDA) and 1,8-diazabicyclo[5.4.0]undec-7-ene (DBU).

3. The process according to any of claims 1 and 2, **characterized in that** the reaction is conducted in a solvent selected from among dimethylformamide (DMF), dimethylsulfoxide (DMSO), tetrahydrofuran (THF), 2-methyl-tetrahydrofuran (MeTHF) and dimethyl ether (DME).

4. The process according to any of claims 1 to 3, **characterized by** a (II):(III) weight ratio of about 1:1.

5. The process according to any of claims 1 to 4, **characterized in that** the compound of following formula (IV): 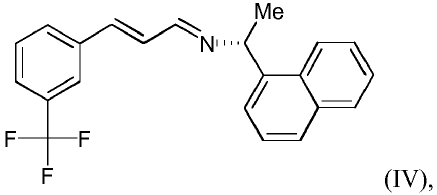 obtained by reaction of the compounds of formula (II) and (III),
is hydrogenated to give cinacalcet, the cinacalcet optionally being converted to a pharmaceutically acceptable salt.

6. The process according to claim 5, **characterized in that** hydrogenation is performed in a hydrogen atmosphere in the presence of a hydrogenation catalyst.

7. The process according to claim 6, **characterized in that** the hydrogenation catalyst is palladium on carbon (Pd/C).

8. The process according to any of claims 6 and 7, **characterized in that** the hydrogenation reaction is conducted in ethanol or methanol as solvent.

9. The process according to any of claims 1 to 8, **characterized in that** the pharmaceutically acceptable salt of cinacalcet is the hydrochloride of cinacalcet or the phosphate salt of cinacalcet.

10. A phosphorus-containing derivative of following formula (III): 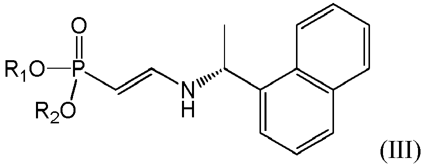 where R₁ and R₂ are such as defined in claim 1.

11. The use of a phosphorus-containing derivative of formula (III) according to claim 10 as synthesis intermediate in a process for preparing cinacalcet or one of the pharmaceutically acceptable salts thereof.

12. The process for preparing a phosphorus-containing derivative of formula (III) according to claim 10, comprising the reaction of the phosphonate derivative of following formula (V): 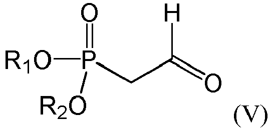 where R₁ and R₂ are such as defined in claim 1,
with (R)-(+)-1-(1-naphthyl)ethylamine of following formula (VI):
